# EUROPÄISCHE PATENTSCHRIFT

(11) **EP 0 782 456 B1**
(45) Veröffentlichungstag und Bekanntmachung des Hinweises auf die Patenterteilung: **12.12.2001**
(21) Anmeldenummer: 95932618.2
(22) Anmeldetag: 22.09.1995
(51) Int. Cl.: A61K 47/48, A61K 41/00

(54) **KONJUGAT AUS EINEM WIRKSTOFF UND EINEM NICHT ALS KÖRPERFREMD ANGESEHENEN, NATIVEN PROTEIN**
CONJUGATE CONSISTING OF AN ACTIVE SUBSTANCE AND A NON-EXOGENOUS NATIVE PROTEIN
CONJUGUE COMPOSE D'UNE SUBSTANCE ACTIVE ET D'UNE PROTEINE NATURELLE CONSIDEREE COMME NON EXOGENE

(30) Priorität: 22.09.1994 DE 4433890
(43) Veröffentlichungstag der Anmeldung: 09.07.1997
(73) Patentinhaber: Deutsches Krebsforschungszentrum Stiftung des öffentlichen Rechts, 69120 Heidelberg (DE)
(72) Erfinder: SINN, Hansjörg, D-69168 Wiesloch (DE); SCHRENK, Hans-Hermann, D-67278 Zeiskam (DE); MAIER-BORST, Wolfgang, D-69221 Dossenheim (DE); STEHLE, Gerd, D-68305 Mannheim (DE); WUNDER, Andreas, D-69214 Eppelheim (DE); HOFF-BIEDERBECK, Dirk, D-67063 Ludwigshafen (DE); HEENE, Dieter, Ludwig, D-68259 Mannheim (DE)
(74) Vertreter: Schüssler, Andrea, Dr.
(86) Internationale Anmeldenummer: DE9501323
(87) Internationale Veröffentlichungsnummer: WO9609071

(56) Entgegenhaltungen:
- WO-A-91/18012
- WO-A-94/27641
- BE-A- 882 541
- DE-A- 4 122 210
- J. PHOTOCHEM. PHOTOBIOL. B: BIOLOGY, Bd. 26, Oktober 1994 Seiten 45-56, HAMBLIN M.R. ET AL. 'PHOTOSENSITIZER TARGETING IN PHOTODYNAMIC THERAPY. I CONJUGATES OF HAEMATOPORPHYRIN WIT ALBUMIN AND TRANSFERRIN.'
- CHEMICAL ABSTRACTS, vol. 115, no. 7, 19.August 1991 Columbus, Ohio, US; abstract no. 67675, HENRIKSEN, ULLA ET AL 'Azidobenzoyl-, azidoacridinyl-, diazocyclopentadienylcarbonyl- and 8-propyloxypsoralen photobiotinylation reagents. Syntheses and photoreactions with DNA and protein' & J. PHOTOCHEM. PHOTOBIOL., A (1991), 57(1-3), 331-42 CODEN: JPPCEJ;ISSN: 1010-6030, 1991

## Beschreibung

Die Erfindung betrifft ein Konjugat aus einem Wirkstoff und einem nicht als körperfremd angesehenen, nativen Protein, Verfahren zur Herstellung eines solchen Konjugates sowie dessen Verwendung.

WO-A-91/18012 beschreibt ein Konjugat aus einem Wirkstoff, z.B. einem Chemotherapeutikum, und einem Peptid, wobei zwischen diesen ein in einer Zelle spaltbarer Linker umfassend eine Disulfidbindung vorliegt.

BE-A-882 541 beschreibt ein Konjugat aus einem Wirkstoff, z.B. einer anti-Tumor-Verbindung, und einem Protein, z.B. einem Immunglobulin, wobei zwischen diesen ein Spacerarm mit einer Peptidstruktur vorliegt.

Chemical Abstracts, Vol 115, No. 7 (1991) beschreibt Verbindungen aus einem photoaktiven Liganden und Biotin, die über einen Azopolymethin-Linker verbunden sind. Diese Verbindungen werden zur Photobiotinylierung von Proteinen verwendet.

Seit langem ist es ein großes Bedürfnis, Arzneimittel gezielt an bestimmte Orte im Körper zu bringen und sie dort ihre Aktivität entfalten zu lassen. Ersteres ist durch ein vorstehendes Konjugat erreicht (vgl. DE - 41 22 210). Mit diesem kann eine tumoraktive Verbindung im Tumor angereichert werden.

Überraschenderweise hat sich nun gezeigt, daß ein vorstehendes Konjugat auch eine sehr hohe Aktivität aufweist, wenn zwischen dem Wirkstoff und dem nicht als körperfremd angesehenen, nativen Protein ein in einer Zelle spaltbarer Linker vorliegt, der eine Azo-Gruppe umfaßt.

Ein derartiges Konjugat ist Gegenstand der vorliegenden Erfindung.

Der vorstehende Ausdruck "Wirkstoff" umfaßt Verbindungen jeglicher Art, die zur Therapie einer Erkrankung verwendet werden können. Dies sind z.B. Verbindungen zur Therapie von Tumor-, Infektions- und/oder Autoimmunerkrankungen. Beispiele solcher Verbindungen sind Chemotherapeutika, wie Antibiotika, z.B. Tetracycline, und Antimetaboliten, z.B. Methotrexat, Sulfonamide und Nukleoside, die nach Einbau in eine Nukleinsäure deren Replikation bzw. Transkription inhibieren. Bevorzugte Verbindungen vorstehender Art sind solche, die eine Säuregruppe, wie -CO₂H, -SO₃H, -PO₃H₂ oder -AsO₃H₂, aufweisen. Besonders bevorzugte Verbindungen sind 4- und 2-Aminobenzoesäure, 4- und 2-Aminophenylsulfonsäure, 4- und 2-Aminophenylphosphonsäure, 4- und 2-Aminophenylarsonsäure sowie Derivate davon. Weiter bevorzugte Verbindungen sind Desoxyuridin (UDR), Desoxycytidin (CDR), Cytosinarabinosid (AraC), 5-Fluoruracil (5-FU), 5-Fluordesoxyuridin(5-FUDR), Azidothymidin (AZT).

Weitere Beispiele von Verbindungen als Wirkstoff sind photoaktive Substanzen, wie Porphyrine, Chlorine und Bakteriochlorine, die zur photodynamischen Therapie verwendet werden können.

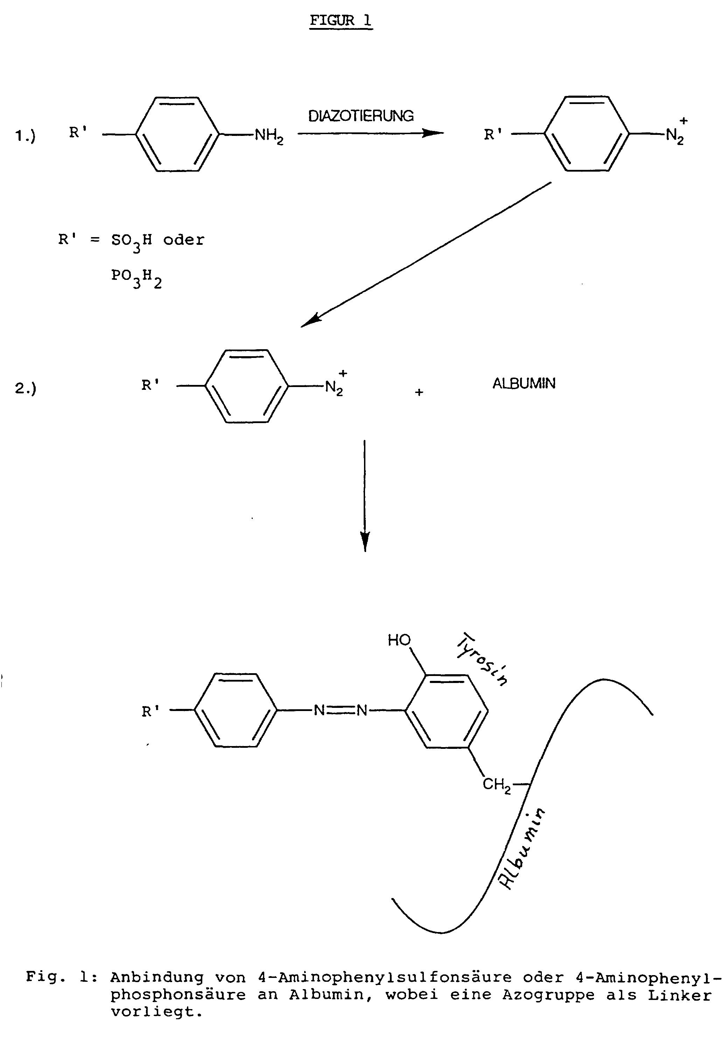

Vorstehende Verbindungen liegen einzeln oder zu mehreren in einem erfindungsgemäßen Konjugat vor. Sie sind als Edukte dargestellt, was bedeutet, daß sie in einem erfindungsgemäßen Konjugat in derivatisierter Form vorliegen (vgl. nachstehend, Beispiele 1-7 und Figuren 1-3).

Ein vorstehender Wirkstoff ist über einen Linker an ein Protein gebunden. Dieses Protein wird vom Körper nicht als fremd angesehen. Auch liegt es in nativer, d.h. nicht-modifizierter, Form vor. Desweiteren hat das Protein ein Molekulargewicht (MG) von bis zu 90000, vorzugweise ist es Albumin, insbesondere humanes Serumalbumin, oder Transferrin.

Ein vorstehender Linker ist in einer Zelle spaltbar. Der Ausdruck "Zelle" umfaßt Einzelzellen und Zellverbände. Beispiele ersterer sind körpereigene, nicht in einem Verband vorliegende Zellen, z.B. Blutzellen und Virus-befallene Zellen, und körperfremde Zellen, z.B. Mikroorganismen, wie Bakterien, Pilze und Protozoen. Zellverbände umfassen Gewebe, Organe und Tumoren.

Ein Linker vorstehender Art ist dem Fachmann bekannt. Auch weiß er um Faktoren, z.B. Enzyme, die in Zellen die Spaltung bestimmter chemischer Bindungen bedingen. Somit ist er in der Lage, weitere Linker zu konstruieren, die in einer Zelle spaltbar sind. Ein solcher Linker umfaßt eine Azo-Gruppe. Diese wird bevorzugt gespalten. Besonders günstig ist es, wenn der Linker folgende Struktur aufweist:

-Y-R-N=N-

worin
R eine organische Gruppe, vorzugsweise eine aromatische, und besonders bevorzugt Phenylen oder ein Derivat davon ist, und
Y eine aus C(O), S(O)₂, P(O)OH und As(O)OH ausgewählte Gruppe ist.

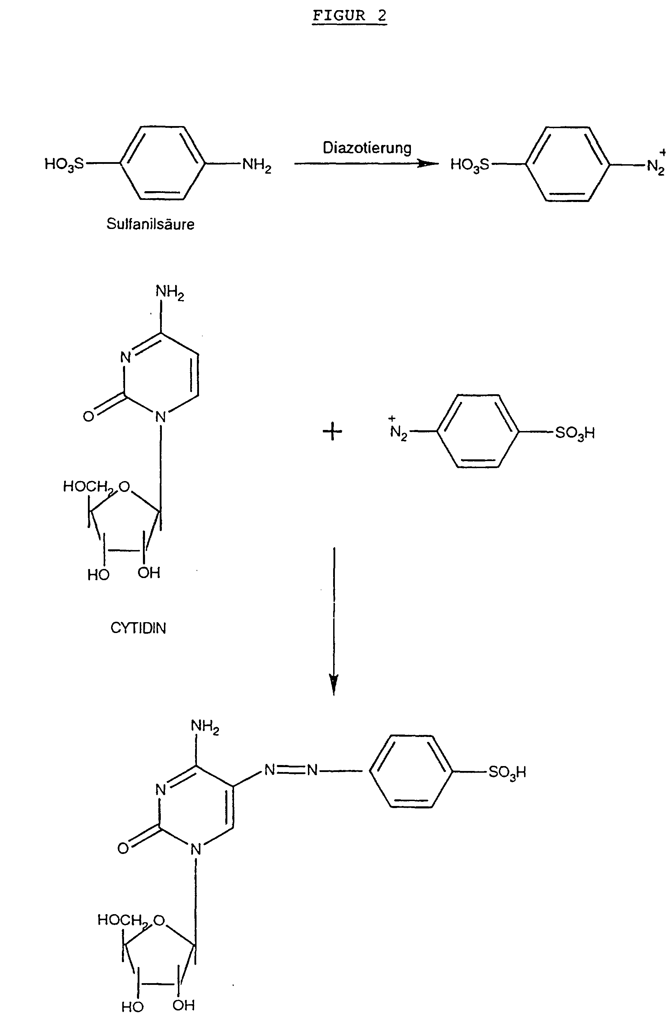

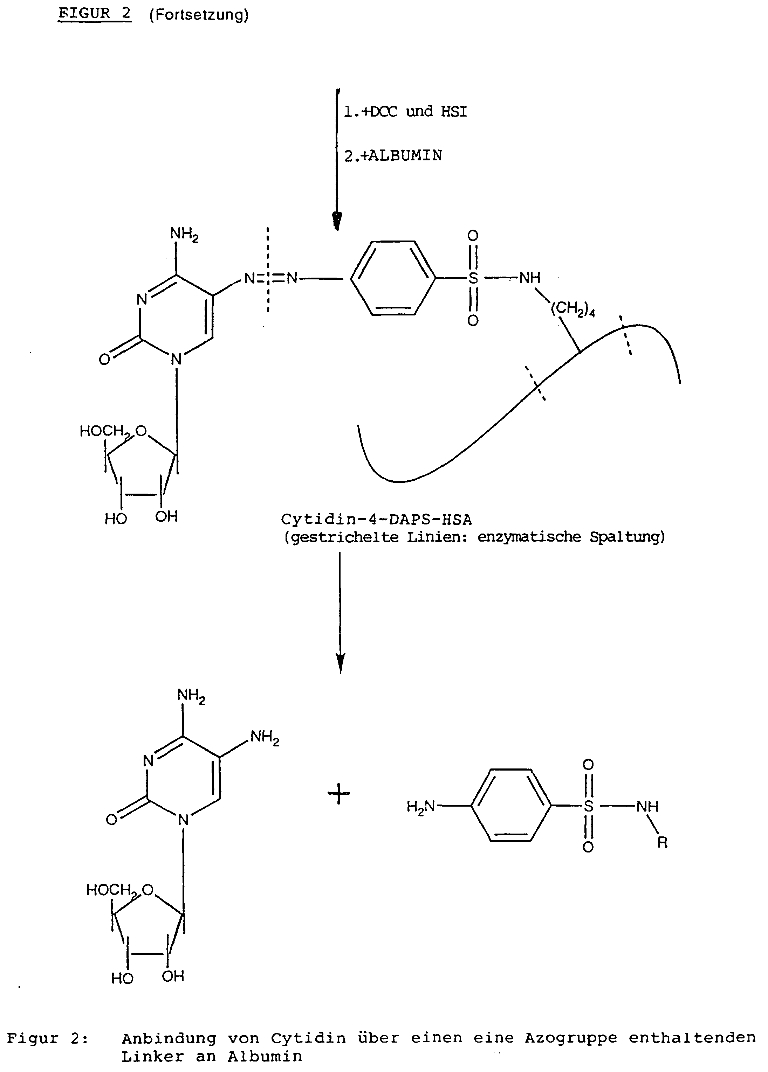

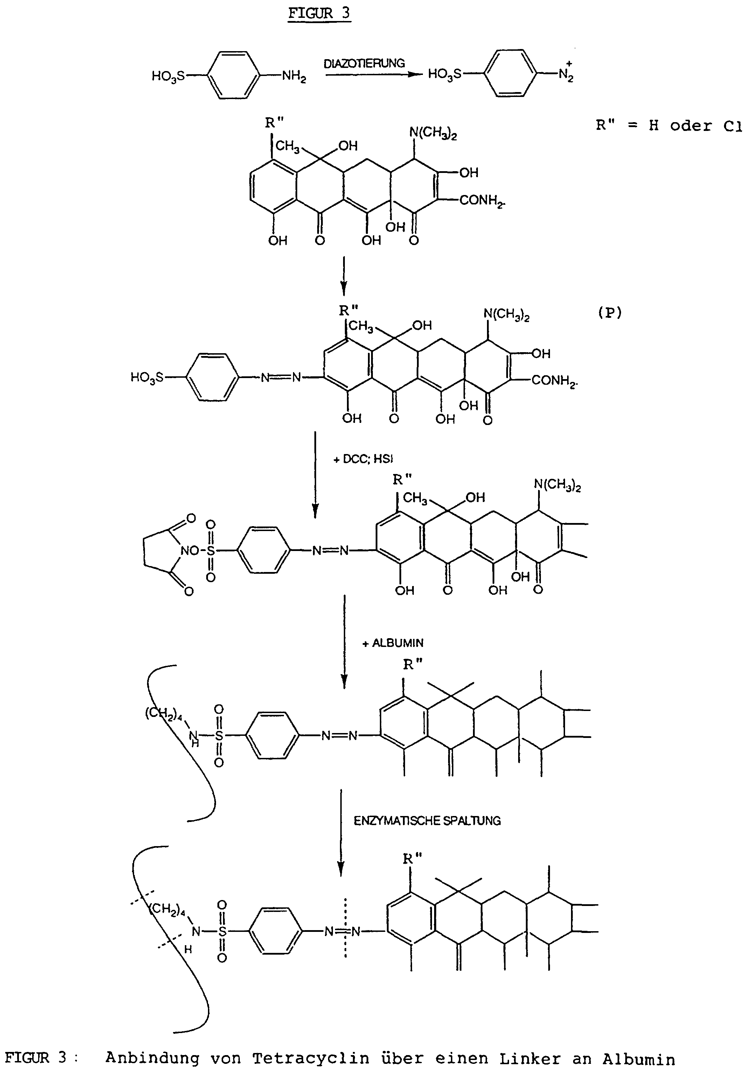

Vorstehende Struktur eines bevorzugten Linkers entspricht jener, die der Linker in einem erfindungsgemäßen Konjugat aufweist. Ferner umfaßt die Struktur, zumindest wenn R Phenylen oder ein Derivat davon ist, eine aktive Verbindung, die sich besonders für die Therapie von Tumor-, Infektions- und Autoimmunerkrankungen eignet. Nach Spaltung des Linkers und gegebenenfalls Abbau des noch am Linker gebundenen Proteins kann die Verbindung ihre volle Aktivität entfalten (vgl. nachstehend, Beispiele 3 bis 7 und Figuren 2 und 3).

Bevorzugte Konjugate der vorliegenden Erfindung sind in den Figuren 1-3 angegeben.

Erfindungsgemäß wird auch ein Verfahren zur Herstellung eines vorstehenden Konjugats bereitgestellt. In einem solchen Verfahren werden übliche Umsetzungen der Chemie, wie Diazotierung einer Aminogruppe und Aktivierung einer Säuregruppe einzeln eingesetzt oder kombiniert. Es wird auf die Herstellung der Konjugate in den Beispielen 1-7 und Figuren 1-3 verwiesen.

Erfindungsgemäße Konjugate zeichnen sich dadurch aus, daß sie Wirkstoffe gezielt in bestimmten Zellen des Körpers anreichern und ihnen dort erlauben, ihre Aktivität voll zu entfalten. Dies wird durch die Kombination aus einem Protein, z.B. Albumin, und einem in einer Zelle spaltbaren Linker erreicht. Bestimmte Zellen im Körper, insbesondere Tumorzellen, Zellen entzündlicher Gewebe und Mikroorganismen, nehmen bevorzugt Albumin auf und spalten aufgrund ihrer Enzyme das Linker-Wirkstoff-Konjugat, wodurch der bzw. die Wirkstoff(e) freigesetzt wird (werden) und seine (ihre) Aktivität voll entfalten kann(können).

Erfindungsgemäße Konjugate eignen sich somit bestens für therapeutische Zwekke, insbesondere zur Therapie von Tumor-, Infektions- und Autoimmunerkrankungen.

Desweiteren können in erfindungsgemäßen Konjugaten Markierungen (z.B. radioaktive Markierungen) vorliegen, wodurch die Konjugate auch für diagnostische Zwecke und Therapieüberwanderung, gegebenenfalls gleichzeitig zur Therapie, eingesetzt werden können.

### Kurze Beschreibung der Zeichnungen:

- Fig. 1: zeigt die Anbindung von 4-Aminophenylsulfonsäure oder 4-Aminophenylphosphonsäure an Albumin, wobei eine Azogruppe als Linker vorliegt,
- Fig. 2: zeigt die Anbindung von Cytidin an Albumin, wobei ein eine Azo-Gruppe enthaltender Linker vorliegt, sowie die Freisetzung von Aminocytidin,
- Fig. 3: zeigt die Anbindung von Tetracyclin an Albumin, wobei ein eine Azo-Gruppe enthaltender Linker vorliegt, und
- Fig. 4: zeigt die Wachstumsinhibition von Tumorzellen durch Verabreichung erfindungsgemäßer Konjugate.

Die folgenden Beispiele erläutern die Erfindung.

### Beispiel 1: Herstellung eines erfindungsgemäßen Konjugats aus humanem Serumalbumin und 4-Aminophenylsulfonsäure, wobei eine Azogruppe als Linker vorliegt

Die Herstellung des Konjugats und seine Struktur sind in Fig. 1 gezeigt.

### 1. Diazotierung von 4-Aminophenylsulfonsäure:

4-Aminophenylsulfonsäure (173 mg, 1 mMol) wurden in 5 ml 2 N HCI gelöst. Die Lösung wurde im Eisbad abgekühlt und unter ständigem Rühren wurden 600 *µ*l einer eisgekühlten 2,5 M NaNO₂-Lösung (1,5 mMol) in Portionen à 0,1 ml zugegeben. Nach etwa 10 min wurde der Überschuß an Nitrit durch Zugabe von Harnstoff beseitigt. Es wurde 4-Diazoniumphenylsulfonsäure (4-DAPS) erhalten.

### 2. Kopplung von 4-DAPS an humanes Serumalbumin (HSA):

Zu einer Lösung von 2 g HSA in 30 ml 0,17 M Bic wurde die unter 1. erhaltene 4-DAPS-Lösung in einem molaren Verhältnis von 1 : 1 langsam unter pH-Kontrolle und ständigem Rühren zugegeben, so daß der pH-Wert stets über 7,5 blieb. Schon während der Zugabe von 4-DAPS begann sich die Lösung rot zu färben, wobei die Färbung mit fortschreitender Reaktionsdauer ständig zunahm. Die Abtrennung von Verunreinigungen, wie überschüssiger Harnstoff oder Salze, erfolgte durch Ultrafiltration über eine YM 30 Membran in einer Amicon Druckfiltrationszelle. Es wurde ein Konjugat aus 4-Aminophenylsulfonsäure und HSA erhalten, wobei eine Azogruppe als Linker vorliegt.

Die Reinheit des erfindungsgemäßen Konjugats wurde mittels HPLC (Vorsäule: Zorbax Diol 20 *µ*(50x4mm), Säule 1: Zorbax GF 450, Säule 2: Zorbax GF 250, Laufmittel: 0,2 M Na-citrat, pH 7,5, Fluß: 1 ml/min) überprüft.

### Beispiel 2: Herstellung eines erfindungsgemäßen Konjugats aus humanem Serumalbumin und 4-Aminophenylphosphonsäure, wobei eine Azogruppe als Linker vorliegt

Die Herstellung des Konjugats und seine Struktur sind in Fig. 1 gezeigt.

Die Herstellung erfolgte, wie in Beispiel 1 beschrieben, wobei anstatt 4-Aminophenylsulfonsäure die 4-Aminophenylphosphonsäure verwendet wurde.

### Beispiel 3: Herstellung eines erfindungsgemäßen Konjugats aus Cytidin, einen eine Azogruppe enthaltenden Linker und humanem Serumalbumin (Cytidin-4-DAPS-HSA)

Die Herstellung des Konjugats und seine Struktur sind in Fig. 2 gezeigt.

Das 4-DAPS wurde wie in Beispiel 1 beschrieben hergestellt.

### 1. Kopplung von 4-DAPS an Cytidin:

2,6 mMol Cytidin (etwa 600 mg) wurden in 6 ml 2 N NaOH gelöst und unter Rühren wurde die 4-DAPS-Lösung portionsweise (je 1 ml) zugegeben. Die zunächst farblose Cytidin-Lösung nimmt schon während der Zugabe von 4-DAPS eine zunehmend intensiv rote Färbung an. Nach Vervollständigung der Reaktion wurde die tiefrote Lösung mit 1 N HCI auf einen pH-Wert von etwa 2 eingestellt und anschließend lyophilisiert. Der nach dem Lyophilisieren erhaltene trockene Rückstand wird anschließend in einem Gemisch von 8 ml Methanol und 2 ml DMF gelöst und durch Filtration vom unlöslichen Bodenkörper abgetrennt. Es wurde 5(4-Diazophenylsulfonsäure)-cytidin (5(4-DAPS)-Cytidin) erhalten.

Die Reinheit des Produkts wurde mittels Dünnschichtchromatographie (Platten mit Fluoreszenz-Indikator, Laufmittel: Etac/MeOH 1/1) überprüft.

### 2. Aktivierung von 5(4-DAPS)-Cytidin zum entsprechenden HSI-Ester:

Ein Aliquot der Lösung des 5(4-DAPS)-Cytidin wurde im selben Lösungsmittel (4 Teile Methanol und 1 Teil DMF) mit der doppelten molaren Menge Dicyclohexylcarbodiimid (DCC) und der 7 bis 10-fachen molaren Menge an N-Hydroxysuccinimid (HSI) versetzt. Nach einer Reaktionszeit von etwa 1 h ist die Aktivierung des 5(4-DAPS)-Cytidin zum entsprechenden HSI-Ester beendet. Er kann direkt zur Kopplung an HSA eingesetzt werden.

### 3. Kopplung des HSI-Esters von 5(4-DAPS)-Cytidin an HSA:

Zu einer Lösung von 2 g HSA in 30 ml 0,17 M Bic wurde der HSI-Ester von 5(4-DAPS)-Cytidin langsam unter ständigem Rühren zugegeben. Schon während der Zugabe des HSI-Esters von 5(4-DAPS)-Cytidin fällt DCC aus. Die Trübung von DCC und DC-Harnstoff wurde mittels Filtration abgetrennt. Die Abtrennung anderer Verunreinigungen, wie Methanol, DMF und HSI, erfolgte anschließend über eine YM 30 Membran in einer Amicon Druckfiltrationszelle. Es wurde Cytidin-4-DAPS-HSA erhalten.

Die Reinheit des erfindungsgemäßen Konjugats wurde mittels HPLC (vgl. Beispiel 1) überprüft.

### Beispiel 4: Herstellung eines erfindungsgemäßen Konjugats aus UDR, einem eine Azogruppe enthaltenden Linker und humanem Serumalbumin (UDR-4-DAPS-HSA)

Die Herstellung des erfindungsgemäßen Konjugats erfolgte, wie in Beispiel 3 beschrieben, wobei anstelle von Cytidin UDR verwendet wurde. Es wurde UDR-4-DAPS-HSA erhalten.

### Beispiel 5: Herstellung eines erfindungsgemäßen Konjugats aus AraC, einem eine Azogruppe enthaltenden Linker und humanem Serumalbumin (AraC-4-DAPS-HSA)

Die Herstellung des erfindungsgemäßen Konjugats erfolgte, wie in Beispiel 3 beschrieben, wobei anstelle von Cytidin AraC verwendet wurde. Es wurde AraC-4-DAPS-HSA erhalten.

### Beispiel 6: Herstellung eines erfindungsgemäßen Konjugats aus CDR, einem eine Azogruppe enthaltenden Linker und humanem Serumalbumin (CDR-4-DAPS-HSA)

Die Herstellung des erfindungsgemäßen Konjugats erfolgte, wie in Beispiel 3 beschrieben, wobei anstelle von Cytidin CDR verwendet wurde. Es wurde CDR-4-DAPS-HSA erhalten.

### Beispiel 7: Herstellung eines erfindungsgemäßen Konjugats aus 7-Chlortetracyclin, einem eine Azogruppe enthaltenden Linker und humanem Serumalbumin

Die Herstellung des Konjugats und seine Struktur sind in Fig. 3 gezeigt.

4-DAPS wurde wie in Beispiel 1 beschrieben hergestellt.

### 1. Kopplung von 4-DAPS an 7-Chlortetracyclin:

718,5 mg (1,5 mM) von 7-Chlortetracyclin (MG 478,9) wurden in 20 ml 1 N NaOH gelöst und unter ständigem Rühren die 4-DAPS-Lösung portionsweise (je 1 ml) zugegeben. Die zuerst gelb gefärbt 7-Chlortetracyclin-Lösung nahm während der Zugabe von 4-DAPS eine zunehmend intensiv rote Färbung an. Nach einer Reaktionszeit von etwa 24 h wurde die tiefrote Lösung mit 1 N HCI auf einen pH-Wert von etwa 2 eingestellt und lyophilisiert. Der trockne Rückstand wurde anschließend in einem Gemisch von 8 ml MeOH und 2 ml DMF gelöst und durch Filtration vom unlöslichen Bodenkörper abgetrennt. Es wurde 7-Chlor-9(4-diazophenylsulfonsäure)-tetracyclin (4-DAPS-Chlortetracyclin) erhalten.

### 2. Aktivierung des 4-DAPS-Chlortetracyclins zur Proteinkopplung:

Ein Aliquot der Lösung des 4-DAPS-Tetracyclins wurde im selben Lösungsmittel (4 Teile MeOH und 1 Teil DMF), ohne vorherige Abtrennung des überschüssigen 7-Chlortetracyclins mit der doppelten molaren Menge an DCC (bezogen auf die eingesetzte Menge an Phenylsulfonsäure) und der 7 - 10-fachen molaren Menge an HSI versetzt. Nach einer Reaktionszeit von etwa 2 h ist die Aktivierung des 4-DAPS-Chlortetracyclins zum entsprechenden HSI-Ester beendet. Der so erhaltene Ester kann direkt zur Proteinkopplung eingesetzt werden.

### 3. Kopplung des HSI-Esters von 4-DAPS-Chlortetracyclin an HSA:

Zu einer Lösung von 2g HSA in 30 ml 0,17 M Bic gibt man die äquimolare Menge an HSI-Ester von 4-DAPS-Chlortetracyclin langsam und unter ständigem Rühren. Schon während der Zugabe des HSI-Esters fällt der Überschuß an DCC aus. Die Trübung von DCC und DC-Harnstoff wurde vor der Druckfiltration mittels Filtration abgetrennt. Die Abtrennung anderer Verunreinigungen, wie MeOH, DMF und HSI, erfolgte über eine YM 30 Membran in einer Amicon Druckfiltrationszelle. Es wurde 7-Chlor-9(4-diazophenylsulfonsäure)-tetracyclin-HSA erhalten.

Die Reinheit des so erhaltenen Konjugats wurde mittels HPLC (vgl. Beispiel 1) bestimmt.

### Beispiel 8: Herstellung eines Konjugats aus Tetracyclin, einem eine Azo-gruppe enthaltenden Linker und humanem Serumalbumin

Die Herstellung des Konjugats erfolgte wie in Beispiel 7 beschrieben, wobei anstatt 7-Chlortetracyclin Tetracyclin verwendet wurde. Die Struktur des Konjugats ist in Fig. 3 gezeigt.

### Beispiel 9: Wachstumsinhibition von Tumorzellen durch Verabreichung erfindungsgemäßer Konjugate

Die Konjugate UDR-4-DAPS-HSA (vgl. Beispiel 4), AraC-4-DAPS-HSA (vgl. Beispiel 5) und CDR-4-DAPS-HSA (vgl. Beispiel 6) sowie als Kontrolle HSA alleine wurden jeweils mit Walker-256-Zellen unter üblichen Bedingungen inkubiert. Nach 24, 48 bzw. 72 h wurde in üblicher Weise die Zellzahl pro ml bestimmt.

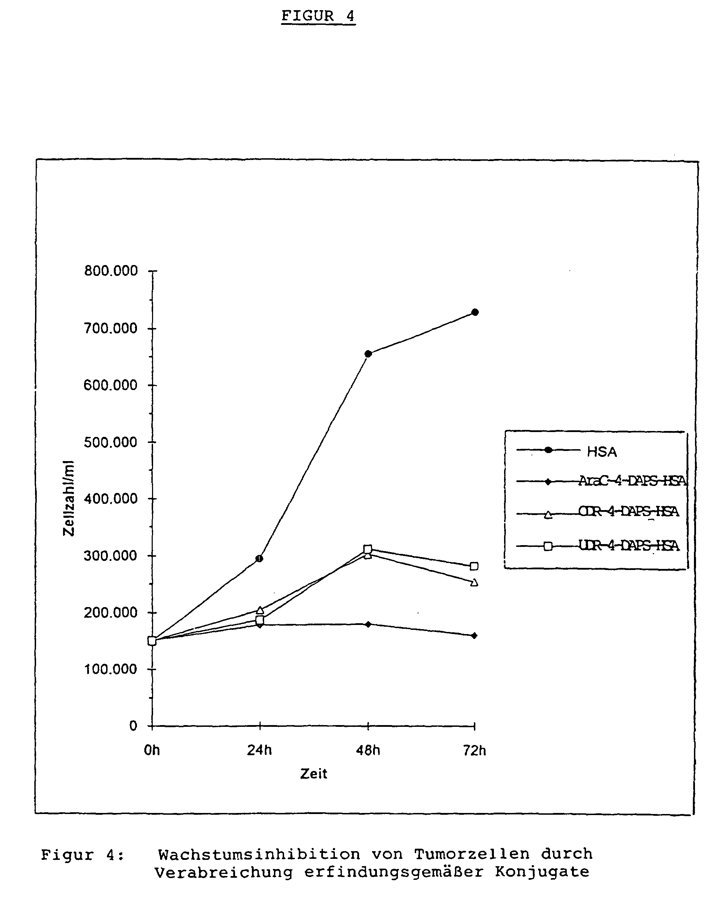

Wie aus Fig. 4 zu ersehen ist, vermindert jedes der erfindungsgemäßen Konjugate im Vergleich zur Kontrolle die Proliferation von Tumorzellen.

## Patentansprüche

1. Konjugat aus einem Wirkstoff und einem nicht als körperfremd angesehenen, nativen Protein, wobei zwischen dem Wirkstoff und dem Frotein ein in einer Zelle spaltbarer Linker vorliegt, **dadurch gekennzeichnet, daß** der Linker eine Azo-Gruppe umfaßt.

2. Konjugat nach Anspruch 1, **dadurch gekennzeichnet, daß** der Wirkstoff eine zur Therapie von Tumor-, Infektions- und/oder Autoimmunerkrankungen verwendbare Verbindung ist.

3. Konjugat nach Anspruch 2, **dadurch gekennzeichnet, daß** der Wirkstoff ein Chemotherapeutikum und/oder eine photoaktive Verbindung ist.

4. Konjugat nach Anspruch 3, **dadurch gekennzeichnet, daß** das Chemotherapeutikum ein Antibiotikum ist.

5. Konjugat nach Anspruch 3, **dadurch gekennzeichnet, daß** das Chemotherapeutikum ein Antimetabolit ist.

6. Konjugat nach einem der Ansprüche 1-5, **dadurch gekennzeichnet, daß** mehrere Wirkstoffe vorliegen.

7. Konjugat nach einem der Ansprüche 1-6, **dadurch gekennzeichnet, daß** der Linker folgende Struktur aufweist:
-Y-R-N = N-
worin
R eine aromatische Verbindung ist, und
Y eine-aus C(O), S(O)₂, P(O)OH und As(O)OH ausgewählte Gruppe ist.

8. Konjugat nach einem der Ansprüche 1-7, **dadurch gekennzeichnet, daß** das Protein Albumin ist.

9. Konjugat nach Anspruch 1, aus humanem Serumalbumin, 4-Aminophenylsulfonsäure oder 4-Aminophenylphosphonsäure, wobei eine Azogruppe als Linker vorliegt.

10. Konjugat nach Anspruch 1, aus humanem Serumalbumin und Cytidin, wobei ein eine Azogruppe aufweisender Linker vorliegt.

11. Konjugat nach Anspruch 1, aus humanem Serumalbumin und 7-Chlortetracyclin, wobei ein eine Azogruppe aufweisender Linker vorliegt.

12. Verfahren zur Herstellung des Konjugates nach Anspruch 1, **dadurch gekennzeichnet, daß** das Anbinden des Wirkstoffs über den Linker an das Protein eine Diazotierung von Aminogruppen und/oder eine Aktivierung von Säuregruppen umfaßt.

13. Verwendung des Konjugats nach einem der Ansprüche 1 bis 11 zur Her stellung einer Zusammensetzung zur Therapie von Tumor-, Infektions und/oder Autoimmunerkrankungen.

## Claims

1. A conjugate consisting of an active substance and a native protein which is not considered foreign to the body, an intracellularly cleavable linker being present between the active substance and the protein, **characterized in that** the linker comprises an azo group.

2. The conjugate according to claim 1, **characterized in that** the active substance is a compound usable for treating tumoral, infectious and/or autoimmune diseases.

3. The conjugate according to claim 2, **characterized in that** the active substance is a chemotherapeutic agent and/or a photoactive compound.

4. The conjugate according to claim 3, **characterized in that** the chemotherapeutic agent is an antibiotic.

5. The conjugate according to claim 3, **characterized in that** the chemotherapeutic agent is an antimetabolite.

6. The conjugate according to any one of claims 1 to 5, **characterized in that** several active substances are present.

7. The conjugate according to any one of claims 1 to 6, **characterized in that** the linker has the following structure:
-Y-R-N=N-
wherein
R is an aromatic compound, and
Y is a group selected from C(O), S(O)₂, P(O)OH and As(O)OH.

8. The conjugate according to any one of claims 1 to 7, **characterized in that** the protein is albumin.

9. The conjugate according to claim 1, from human serum albumin, 4-aminophenylsulfonic acid or 4-aminophenylphosphonic acid, wherein an azo group is present as the linker.

10. The conjugate according to claim 1, from human serum albumin and cytidine, wherein a linker having an azo group is present.

11. The conjugate according to claim 1, from human serum albumin and 7-chlorotetracycline, wherein a linker having an azo group is present.

12. A process for the preparation of the conjugate according to claim 1, **characterized in that** the binding of the active substance via the linker to the protein comprises a diazotization of amino groups and/or an activation of acid groups.

13. Use of the conjugate according to any one of claims 1 to 11 for the production of a composition for treating tumoral, infectious and/or autoimmune diseases.

## Revendications

1. Conjugué d'une substance active et d'une protéine native considérée comme non étrangère à l'organisme, dans lequel il existe entre la substance active et la protéine un segment de liaison pouvant être clivé dans une cellule, **caractérisé en ce que** le segment de liaison comprend un groupe azoïque.

2. Conjugué suivant la revendication 1, **caractérisé en ce que** la substance active est un composé utilisable pour la thérapie de maladies tumorales, de maladies infectieuses et/ou de maladies auto-immunes.

3. Conjugué suivant la revendication 2, **caractérisé en ce que** la substance active est un composé chimiothérapeutique et/ou un composé photo-actif.

4. Conjugué suivant la revendication 3, **caractérisé en ce que** le composé chimiothérapeutique est un antibiotique.

5. Conjugué suivant la revendication 3, **caractérisé en ce que** l'agent chimiothérapeutique est un antimétabolite.

6. Conjugué suivant l'une des revendications 1 à 5, **caractérisé en ce que** plusieurs substances actives sont présentes.

7. Conjugué suivant l'une des revendications 1 à 6, **caractérisé en ce que** le segment de liaison présente la structure suivante :
-Y-R-N=N-
dans laquelle
R est un composé aromatique, et
Y est un groupe choisi entre C(O), S(O)₂, P(O)OH et As(O)OH.

8. Conjugué suivant l'une des revendications 1 à 7, **caractérisé en ce que** la protéine consiste en l'albumine.

9. Conjugué suivant la revendication 1, constitué de sérum-albumine humaine, d'acide 4-aminophénylsulfonique ou d'acide 4-aminophénylphosphonique, dans lequel un groupe azoïque est présent comme segment de liaison.

10. Conjugué suivant la revendication 1, constitué de sérum-albumine humaine et de cytidine, dans lequel un segment de liaison présentant un groupe azoïque est présent.

11. Conjugué suivant la revendication 1, constitué de sérum-albumine humaine et de 7-chlorotétracycline, dans lequel un segment de liaison présentant un groupe azoïque est présent.

12. Procédé de préparation du conjugué suivant la revendication 1, **caractérisé en ce que** la liaison de la substance active à la protéine par l'intermédiaire du segment de liaison comprend une diazotation de groupes amino et/ou une activation de groupes acides.

13. Utilisation du conjugué suivant l'une des revendications 1 à 11 pour la préparation d'une composition destinée à la thérapie de maladies tumorales, de maladies infectieuses et/ou de maladies auto-immunes.
